# EUROPÄISCHE PATENTANMELDUNG

(11) **EP 4 371 504 A1**
(43) Veröffentlichungstag der Anmeldung: **22.05.2024**
(21) Anmeldenummer: 23209595.0
(22) Anmeldetag: 14.11.2023
(51) Int. Cl.: A61B 17/15

(54) **RESEKTIONSSCHABLONE ZUR RESEKTION VON EINEM KNOCHEN**

(30) Priorität: 18.11.2022 DE 102022212308
(71) Anmelder: Karl Leibinger Medizintechnik GmbH & Co. KG, 78570 Mühlheim/Donau (DE)
(72) Erfinder: LEIBINGER, Ralf, 78570 Mühlheim (DE); KÜHN, Artur, 78315 Radolfzell (DE); REINAUER, Frank, 78576 Emmingen-Liptingen (DE)
(74) Vertreter: Isarpatent

(57) **Zusammenfassung**

Die Erfindung schafft eine Resektionsschablone zur Resektion von einem Knochen. Die Resektionsschablone ist ausgestattet mit einem plattenförmigen Basisteil (1) mit einer Vorderseite (VS) und einer Rückseite (RS), wobei die Rückseite (RS) eine Auflagefläche (10) zur Auflage auf dem Knochen aufweist, wobei die Vorderseite (VS) einen sich von der Vorderseite (VS) weg erstreckenden Befestigungsflansch (2) aufweist, welcher benachbart zu einer Endkante (K) des Basisteils (1) oder einem Schlitz des Basisteils (1) angeordnet ist, einem Schnittführungsaufsatz (20) , welcher von einer der Vorderseite (VS) gegenüberliegenden Seite (S2) des Befestigungsflansches (2) auf den Befestigungsflansch (2) aufsteckbar ist, wobei der Schnittführungsaufsatz (20) einen Sägeschlitz (201) oder einen Sägeanschlag zum Führen eines Sägeschnittwerkzeugs aufweist, welcher bei aufgestecktem Schnittführungsaufsatz (20) entlang der Endkante (K) des Basisteils (1) oder entlang des Schlitzes des Basisteils (1) angeordnet ist.

## Beschreibung

Die vorliegende Erfindung betrifft eine Resektionsschablone zur Resektion von einem Knochen.

Obwohl für beliebige Knochen anwendbar, werden die vorliegende Erfindung und die ihr zugrundeliegende Problematik anhand vom Knochen im craniomaxillfacialen Bereich und Knochenextremitäten erläutert.

Resektionsschablonen können generell bei allen chirurgischen Osteotomien verwendet werden, werden aber besonders häufig bei der Rekonstruktion des Unterkiefers mit einem autologen Knochentransplantat (Fibula oder Beckenkamm) oder bei der Resektion von Meningeomen eingesetzt.

Trotz der Fortschritte mikrovaskulärer Rekonstruktionstechniken bleibt es eine anspruchsvolle Aufgabe, einen Knochen, z.B. den Unterkieferknochen, in seine ursprüngliche anatomische Form zurückzubringen und funktionelle und ästhetische Aspekte wiederherzustellen. Als bewährtes Verfahren zur Wiederherstellung, hat sich die Verwendung einer Fibula vom Unterschenkel des Patienten etabliert. Aber auch andere Spender-Regionen sind in dem Zusammenhang als Transplantat zu nennen, wie z.B. die Entnahme von Knochensegmenten an Beckenkamm oder Scapula.

Produktverbesserungen der vergangenen Jahre zielen hauptsächlich auf computergestützte Ansätze in Kombination mit patientenspezifisch angefertigten Resektionsschablonen und Implantaten ab, mithilfe derer die präoperative Planung in die Echtzeit-Chirurgie übertragen werden kann.

Entscheidend für die korrekte Umsetzung dieser Planungen bei der Operation ist eine möglichst präzise Osteotomie/Resektion am zu verwendenden Transplantat/Resektat. Dies kann nur mit einer definierten Schnittführung und exakten Schnittflächen am Cutting Guide erreicht werden.

Die bisherigen Verfahren stützen sich weitestgehend auf dem Prinzip eines metallischen Inlays, welches in eine Umrandung aus Kunststoff eingesetzt wird. Daraus resultiert aber eine entsprechend groß dimensionierte Baugruppe, welche einer sehr großen Wundöffnung bedarf und somit eine erhebliche Belastung für den Patienten darstellt, da der Einschub des Inlays lateral erfolgt.

Die EP 3 451 944 A1 offenbart eine Fibula-Knochenmaterialentnahme- und -verbringschablone mit einem Mittelteil, das einen Zentralkörper besitzt, an dessen Enden je ein Knochentrennwerkzeugführabschnitt vorhanden ist, wobei zumindest einer der Knochentrennwerkzeugführabschnitte vom Mittelteil entfernbar und heranschiebbar gelagert ist.

Die DE 10 2016 108 433 A1 offenbart eine Unterkieferresektionsschablone mit einem Zentralbauteil, das zum Anbringen an einem Segment, etwa einem Symphysensegment eines Kieferknochens, wie eines Unterkiefers oder eines Oberkiefers vorbereitet ist, wobei an dem Zentralbauteil mindestens zwei Trennwerkzeugführabschnitte vorhanden sind, wobei zwischen den beiden oder mehreren Trennwerkzeugführabschnitten eine Positionierhilfe vorhanden ist, um eine raumkorrekte Ausrichtung der Unterkieferresektionsschablone zum Kieferknochen zu bewerkstelligen.

Die US 2021/0137537 A1 offenbart eine Resektionsschablone, umfassend eine Schneidführung, die mehrere Schneidschlitze definiert und mindestens einen entfernbaren Einsatz umfasst, der zwischen den mehreren Schneidschlitzen positioniert ist, wobei der mindestens eine entfernbare Einsatz eine Öffnung definiert, die dazu ausgelegt ist, eine chirurgische Befestigungsvorrichtung aufzunehmen; und
eine Schablonenführung, die ein Merkmal definiert, wobei der mindestens eine entfernbare Einsatz so gestaltet ist, dass er zumindest teilweise unter Verwendung des Merkmals in die Schablonenführung passt.

Weitere Resektionsschablonen sind aus der WO 2017/162444 A1 und der EP 3 470 002 A1 bekannt.

Es ist die Aufgabe der vorliegenden Erfindung, eine verbesserte Resektionsschablone zur Resektion von einem Knochen zu schaffen, welche intraoperativ besser zugänglich ist und einen geringeren Platzbedarf hat.

Die Aufgabe wird durch eine Resektionsschablone zur Resektion von einem Knochen mit den Merkmalen des Anspruchs 1 gelöst, also durch eine Resektionsschablone zur Resektion von einem Knochen mit einem plattenförmigen Basisteil mit einer Vorderseite und einer Rückseite, wobei die Rückseite eine Auflagefläche zur Auflage auf dem Knochen aufweist, wobei die Vorderseite einen sich von der Vorderseite weg erstreckenden Befestigungsflansch aufweist, welcher benachbart zu einer Endkante des Basisteils oder einem Schlitz des Basisteils angeordnet ist, einem Schnittführungsaufsatz, welcher von einer der Vorderseite gegenüberliegenden Seite des Befestigungsflansches auf den Befestigungsflansch aufsteckbar ist, wobei der Schnittführungsaufsatz einen Sägeschlitz oder einen Sägeanschlag zum Führen eines Sägeschnittwerkzeugs aufweist, welcher bei aufgestecktem Schnittführungsaufsatz entlang der Endkante des Basisteils oder entlang des Schlitzes des Basisteils angeordnet ist.

Gemäß der Erfindung zugrundeliegenden Idee wird die Aufnahme wird mehr als Umrandung oder Rahmen auf einem Grundkörper realisiert, sondern durch einen von der Oberseite ausgehenden vorzugsweise schlanken Befestigungsflansch, z.B. Plattenflansch oder Dornflansch, der die gesamte Konstruktion in einer weitaus geringeren Baugröße ermöglicht. Somit kann mit einer deutlich kleineren patientenschonenden Wundöffnung operiert werden.

Die Erfindung basiert auf einem Basiskörper aus einem körperverträglichen Material, z.B. einem Kunststoff, und einem Schnittführungsaufsatz mit einer definierten Positionierung und Schnittführung aus einem widerstandsfähigen Material, z.B. einem Metall, wobei eine offene oder eine geschlossene Variante beim Schnittführungsaufsatz möglich ist. Im Unterschied zu bisher bekannten Systemen, ist die Erfindung nicht als Einlegeteil ausgelegt, sondern als Schnittführungsaufsatz für einen Befestigungsflansch gestaltet.

Der Basiskörper wird auf den zu durchtrennenden Knochen aufgelegt und daran befestigt, z.B. angeschraubt. Der Schnittführungsaufsatz wird dann vom Chirurgen auf den dafür vorgesehenen Befestigungsflansch aufgesteckt. Das Handling geschieht recht einfach mit Daumen und Zeigefinger. Ein vorzugsweise codierter Formschluss lässt nur eine, durch die Planung vorbestimmte, Positionierung zu. Je stärker der Schnittführungsaufsatz auf das Basisteil gedrückt wird, desto mehr Selbsthalt erfährt diese Kombination. Erreicht werden kann dies durch eine optionale Formschräge am Befestigungsflansch, welche sich in der parallel verlaufenden Führung des Schnittführungsaufsatzes verklemmt. Außerdem erzeugt die zur Osteotomie verlagerte Befestigung ein Moment, welches den Guide verkantet und gegen Verlieren - welche durch die Säge und Vibrationen entsteht - sichert.

Die Art der Konstruktion bringt eine signifikante Verringerung der Baugröße mit sich, was im Vergleich zu den bisher bekannten Verfahren einen Vorteil für den Anwender, aber (und vor allem auch) für den Patienten darstellt. Das einfache Design ermöglicht ein problemloses Handling.

Bevorzugte Weiterbildungen der erfindungsgemäßen Gegenstände ergeben sich aus den Unteransprüchen sowie aus der Beschreibung, insbesondere mit Bezug auf die Zeichnungen.

Gemäß einer bevorzugten Ausführungsform ist der Befestigungsflansch als Plattenflansch ausgebildet. Eine derartige Ausgestaltung ist stabil und benötigt wenig Platz.

Gemäß einer weiteren bevorzugten Ausführungsform ist der Schnittführungsaufsatz als Ringprofil ausgebildet, welches einen geradlinigen Bereich aufweist, in dem der Sägeschlitz oder an dem der Sägeanschlag angeordnet ist, wobei sich an dem ersten Ende des geradlinigen Bereichs ein erster Endumschlagbereich und an dem zweiten Ende des geradlinigen Bereichs ein zweiter Endumschlagbereich befindet, wobei der erste und zweite Endumschlagbereich zusammen mit dem geradlinigen Bereich einen Aufnahmebereich für den Plattenflansch bilden, und wobei der erste und zweite Endumschlagbereich derart ausgebildet sind, dass sie den Plattenflansch an dessen gegenüberliegenden Randbereichen umschließen.

Gemäß einer weiteren bevorzugten Ausführungsform befindet sich zwischen dem ersten und zweiten Endumschlagbereich ein offener Bereich des Ringprofils.

Gemäß einer weiteren bevorzugten Ausführungsform weist der Plattenflansch einen ersten Anschlagssteg und einen zweiten Anschlagssteg auf, welche derart angeordnet sind, dass sie einen jeweiligen lateralen Anschlag für den ersten uns zweiten Endumschlagbereich bilden. Dies ermöglicht eine Codierung zur Verhinderung eines fehlerhaften Aufsteckens.

Gemäß einer weiteren bevorzugten Ausführungsform ist der Plattenflansch einen Anschlagsblock aufweist, welcher derart angeordnet, dass er einen jeweiligen lateralen Anschlag für den ersten und zweiten Endumschlagbereich bildet. Dies ermöglicht ebenfalls eine Codierung zur Verhinderung eines fehlerhaften Aufsteckens.

Gemäß einer weiteren bevorzugten Ausführungsform weist der Plattenflansch an seinen gegenüberliegenden Kanten einen jeweiligen ersten und zweiten Verbreiterungsbereich auf, welcher sich in Richtung von der der Vorderseite gegenüberliegenden Seite des Plattenflansches zur Vorderseite hin verbreitert, so dass beim Aufstecken eine zunehmende Klemmwirkung auf das Ringprofil ausübbar ist. Dies sorgt für eine stabile Halterung des Ringprofils.

Gemäß einer weiteren bevorzugten Ausführungsform weist der Plattenflansch an seinen gegenüberliegenden Kanten einen jeweiligen ersten und zweiten Endanschlagsbereich auf, welche die Aufstecktiefe des Ringprofils auf den Plattenflansch begrenzen.

Gemäß einer weiteren bevorzugten Ausführungsform weisen der erste und zweite Endumschlagbereich einen jeweiligen Griffriefenbereich auf. Dies ermöglicht eine sichere Handhabbarkeit.

Gemäß einer weiteren bevorzugten Ausführungsform weist der Plattenflansch einen ersten Schlitz und einen zweiten Schlitz ausgehend von der der Vorderseite gegenüberliegenden Seite des Plattenflansches auf, welche in einem jeweils zugehörigen ersten Verbreiterungsbereich und zweiten Verbreiterungsbereich enden, und wobei der erste und zweite Endumschlagbereich einen ersten und zweiten Verriegelungsbolzen aufweisen, welche durch den zugehörigen Schlitz in den ersten und zweiten Verbreiterungsbereich zum Verriegeln des Ringprofils einführbar sind.

Gemäß einer weiteren bevorzugten Ausführungsform weist der Plattenflansch einen ersten Schlitz und einen zweiten Schlitz ausgehend von der der Vorderseite gegenüberliegenden Seite des Plattenflansches auf, welche in einem jeweils zugehörigen ersten Verbreiterungsbereich und zweiten Verbreiterungsbereich enden, und wobei der geradlinige Bereich einen ersten und zweiten Verriegelungsbolzen aufweist, welche durch den zugehörigen Schlitz in den ersten und zweiten Verbreiterungsbereich zum Verriegeln des Ringprofils einführbar sind.

Gemäß einer weiteren bevorzugten Ausführungsform ist der Befestigungsflansch als Dornflanschflansch mit einem oder mehreren Dornen ausgebildet.

Gemäß einer weiteren bevorzugten Ausführungsform ist der Schnittführungsaufsatz als Ringprofil ausgebildet, welches einen oder mehrere Dornaufnahmebereiche entsprechend der Anzahl von Dornen des Dornflansches aufweist.

Gemäß einer weiteren bevorzugten Ausführungsform weist der Plattenflansch an der der Vorderseite gegenüberliegenden Seite des Plattenflansches eine Nut auf, wobei Schnittführungsaufsatz ausgehend von dem geradlinigen Bereich einen Verbindungsbereich aufweist, welcher in die Nut einsetzbar ist, und wobei an dem geradlinigen Bereich gegenüberliegenden Bereich des Verbindungsbereichs ein Griffbereich vorgesehen ist. Die beiden Griffe können zudem etwas schräg angeordnet sein, um eine Klemmwirkung/ Fixierung zu erzielen. Durch die Flexibilität des Kunststoffs kann dann der Schnittführungsaufsatz durch Zusammendrücken der Griffe herausgenommen werden.

Gemäß einer weiteren bevorzugten Ausführungsform weist das plattenförmigen Basisteil eine Mehrzahl von Durchgangslöchern auf, welche zur Aufnahme von Befestigungsmitteln, insbesondere Schrauben oder Nägeln, zur Befestigung den dem Knochen dienen.

Gemäß einer weiteren bevorzugten Ausführungsform ist das plattenförmige Basisteil mit dem Befestigungsflansch aus einem Kunststoffmaterial und der Schnittführungsaufsatz aus einem metallischen Material gebildet.

Gemäß einer weiteren bevorzugten Ausführungsform ist der Schnittführungsaufsatz derart ausgebildet ist, dass die Oberseite und die Unterseite des Schnittführungsaufsatzes planparallel angeordnet sind, so dass die Schnitttiefe eines aufgesetzten Sägewerkzeuges über die gesamte laterale Erstreckung des Schnittführungsaufsatzes konstant ist.

Gemäß einer weiteren bevorzugten Ausführungsform weist die Oberseite des Schnittführungsaufsatzes eine gekrümmte Kontur auf, welche bewirkt, dass die Schnitttiefe eines aufgesetzten Sägewerkzeuges über die laterale Erstreckung des Schnittführungsaufsatzes variiiert.

Gemäß einer weiteren bevorzugten Ausführungsform ist die gekrümmte Kontur der Oberseite des Schnittführungsaufsatzes derart gekrümmt, dass sie eine Kontur des darunterliegenden zu resektierenden Knochens nachbildet oder zumindest bereichsweise approximiert. Dadurch können durch die Krümmung bestimmte Gewebestrukturen, welche den zu resektierenden Knochen umgeben, geschützt werden, da sie vom Sägewerkzeug nicht erreichbar sind.

Die obigen Ausgestaltungen und Weiterbildungen lassen sich, sofern sinnvoll, beliebig miteinander kombinieren. Weitere mögliche Ausgestaltungen, Weiterbildungen und Implementierungen der Erfindung umfassen auch nicht explizit genannte Kombinationen von zuvor oder im Folgenden bezüglich der Ausführungsbeispiele beschriebenen Merkmalen der Erfindung.

Die Erfindung wird nachstehend anhand von Ausführungsbeispielen in den Figuren der Zeichnung näher erläutert. In teilweise schematisierter Darstellung zeigen hierbei:
- Fig. 1: eine ausschnittsweise perspektivische Darstellung einer Resektionsschablone gemäß einer ersten Ausführungsform der vorliegenden Erfindung;
- Fig. 2: eine ausschnittsweise perspektivische Explosionsansicht der Resektionsschablone gemäß der ersten Ausführungsform der vorliegenden Erfindung;
- Fig. 3: eine ausschnittsweise perspektivische Darstellung des Basisteils der Resektionsschablone gemäß der ersten Ausführungsform der vorliegenden Erfindung;
- Fig. 4: eine perspektivische Darstellung des Schnittführungsaufsatzes der Resektionsschablone gemäß der ersten Ausführungsform der vorliegenden Erfindung;
- Fig. 5a), b): perspektivische Darstellungen einer Resektionsschablone gemäß einer zweiten Ausführungsform der vorliegenden Erfindung, und zwar Fig. 5a) eine ausschnittsweise perspektivische Darstellung mit eingestecktem Sägewerkzeug und Fig. 5b) eine perspektivische Darstellung mit nur einem aufgesteckten Schnittführungsaufsatz;
- Fig. 6: eine perspektivische Darstellung eines Schnittführungsaufsatzes einer Resektionsschablone gemäß einer dritten Ausführungsform der vorliegenden Erfindung;
- Fig. 7: eine perspektivische Darstellung eines Basisteils einer Resektionsschablone gemäß einer vierten Ausführungsform der vorliegenden Erfindung;
- Fig. 8: eine ausschnittsweise perspektivische Explosionsansicht einer Resektionsschablone gemäß einer fünften Ausführungsform der vorliegenden Erfindung;
- Fig. 9: eine ausschnittsweise perspektivische Explosionsansicht einer Resektionsschablone gemäß einer sechsten Ausführungsform der vorliegenden Erfindung;
- Fig. 10: eine ausschnittsweise perspektivische Explosionsansicht einer Resektionsschablone gemäß einer siebenten Ausführungsform der vorliegenden Erfindung;
- Fig. 11: eine ausschnittsweise perspektivische Darstellung einer Resektionsschablone gemäß einer achten Ausführungsform der vorliegenden Erfindung;
- Fig. 12: eine ausschnittsweise perspektivische Darstellung einer Resektionsschablone gemäß einer neunten Ausführungsform der vorliegenden Erfindung; und
- Fig. 13a),b): perspektivische Darstellungen einer Resektionsschablone gemäß einer zehnten Ausführungsform der vorliegenden Erfindung, und zwar Fig. 13a) eine ausschnittsweise perspektivische Darstellung mit eingestecktem Sägewerkzeug in einer ersten Position des Sägewerkzeugs und Fig. 13b) eine ausschnittsweise perspektivische Darstellung mit eingestecktem Sägewerkzeug in einer zweiten Position des Sägewerkzeugs.

In sämtlichen Figuren sind gleiche bzw. funktionsgleiche Elemente und Vorrichtungen - sofern nichts anderes angegeben ist - mit denselben Bezugszeichen versehen worden.

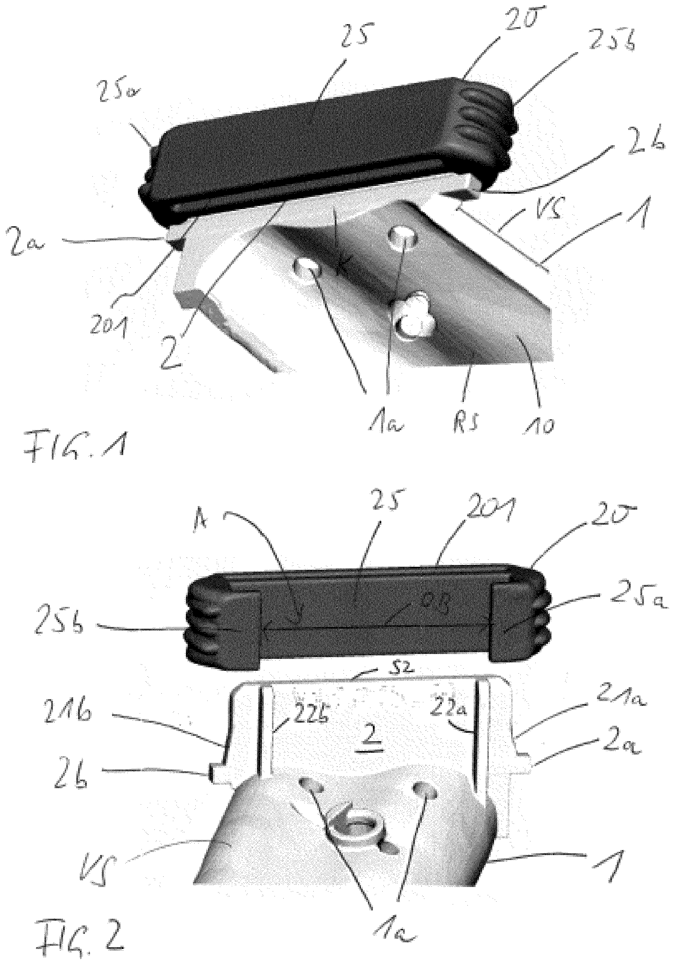

Fig. 1 zeigt eine perspektivische Darstellung einer Resektionsschablone gemäß einer ersten Ausführungsform der vorliegenden Erfindung.

Die Resektionsschablone zur Resektion von einem Knochen gemäß der ersten Ausführungsform weist ein plattenförmiges Basisteil 1 mit einer Vorderseite VS und einer Rückseite RS auf. Es ist aus einem Kunststoffmaterial hergestellt.

Eine Mehrzahl von Durchgangslöchern 1a ist in dem Basisteil 1 vorgesehen, welche zur Aufnahme von Befestigungsmitteln, insbesondere Schrauben oder Nägeln, zur Befestigung an dem Knochen dienen.

Die Rückseite RS weist eine Auflagefläche 10 zur Auflage auf dem Knochen auf, deren Kontur dem zu bearbeitenden Knochen angepasst ist.

Die Vorderseite VS einen sich von der Vorderseite VS weg erstreckenden, intergral mit dem Basisteil ausgebildeten Befestigungsflansch 2 auf, welcher bei dieser Ausführungsform benachbart zu einer Endkante K des Basisteils 1 angeordnet ist.

Ein aus einem metallischen Material gebildeter Schnittführungsaufsatz 20 ist von einer der Vorderseite VS gegenüberliegenden Seite S2 des Befestigungsflansches 2 (vgl. Fig. 2) auf den Befestigungsflansch 2 klemmend aufgesteckt.

Der Schnittführungsaufsatz 20 weist einen Sägeschlitz 201 zum Führen eines Sägeschnittwerkzeugs auf, welcher entlang der Endkante K des Basisteils 1 angeordnet ist.

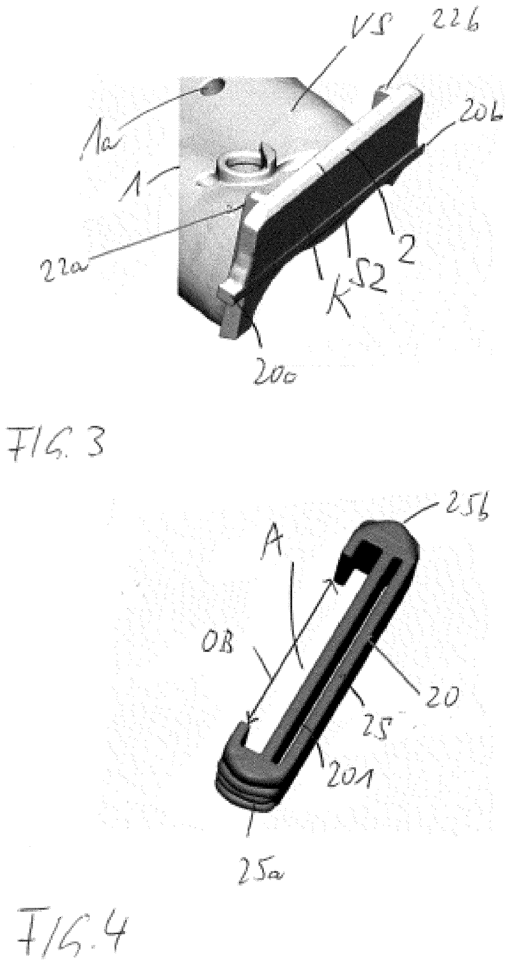

Fig. 2 zeigt eine ausschnittsweise perspektivische Explosionsansicht der Resektionsschablone gemäß der ersten Ausführungsform der vorliegenden Erfindung, Fig. 3 zeigt eine ausschnittsweise perspektivische Darstellung des Basisteils der Resektionsschablone gemäß der ersten Ausführungsform der vorliegenden Erfindung, und Fig. 4 zeigt eine perspektivische Darstellung des Schnittführungsaufsatzes der Resektionsschablone gemäß der ersten Ausführungsform der vorliegenden Erfindung.

Weiter mit Bezug auf Fig. 1 bis 4 ist der Befestigungsflansch 2 als verhältnismäßig schmaler Plattenflansch ausgebildet.

Der Schnittführungsaufsatz 20 ist als offenes Ringprofil ausgebildet, welches einen geradlinigen Bereich 25 aufweist, in dem der Sägeschlitz 201 angeordnet ist, wobei sich an dem ersten Ende des geradlinigen Bereichs 25 ein erster Endumschlagbereich 25a und an dem zweiten Ende des geradlinigen Bereichs 25 ein zweiter Endumschlagbereich 25b befindet, wobei der erste und zweite Endumschlagbereich 25a, 25b zusammen mit dem geradlinigen Bereich 25 einen Aufnahmebereich A für den Plattenflansch bilden. Dazu umschließen der erste und zweite Endumschlagbereich 25a, 25b den Plattenflansch an dessen gegenüberliegenden Randbereichen. Zwischen dem ersten und zweiten Endumschlagbereich 25a, 25b befindet sich ein offener Bereich OB des Ringprofils. Der erste und zweite Endumschlagbereich 25a, 25b einen jeweiligen Griffriefenbereich auf, was ein einfaches Einstecken durch den Chirurgen mittels Daumen und Zeigefinger ermöglicht.

Weiterhin weist der Plattenflansch einen ersten Anschlagssteg 22a und einen zweiten Anschlagssteg 22b auf, welche derart angeordnet sind, dass sie einen jeweiligen lateralen Anschlag für den ersten und zweiten Endumschlagbereich 25a, 25b bilden. Der erste und zweite Anschlagssteg 22a, 22b bildet somit eine Codierung, die ein falsches Aufsetzen des Ringprofils verhindert.

Der Plattenflansch weist an seinen gegenüberliegenden Kanten einen jeweiligen ersten und zweiten Verbreiterungsbereich 21a, 21b auf, welcher sich in Richtung von der der Vorderseite VS gegenüberliegenden Seite S2 des Plattenflansches zur Vorderseite VS hin verbreitert, so dass beim Aufstecken eine zunehmende Klemmwirkung auf das Ringprofil ausgeübt wird.

An den gegenüberliegenden Kanten des Plattenflansches ist ein jeweiliger unterer erster und zweiter Endanschlagsbereich 2a, 2b vorgesehen, welche die Aufstecktiefe des Ringprofils auf den Plattenflansch begrenzen.

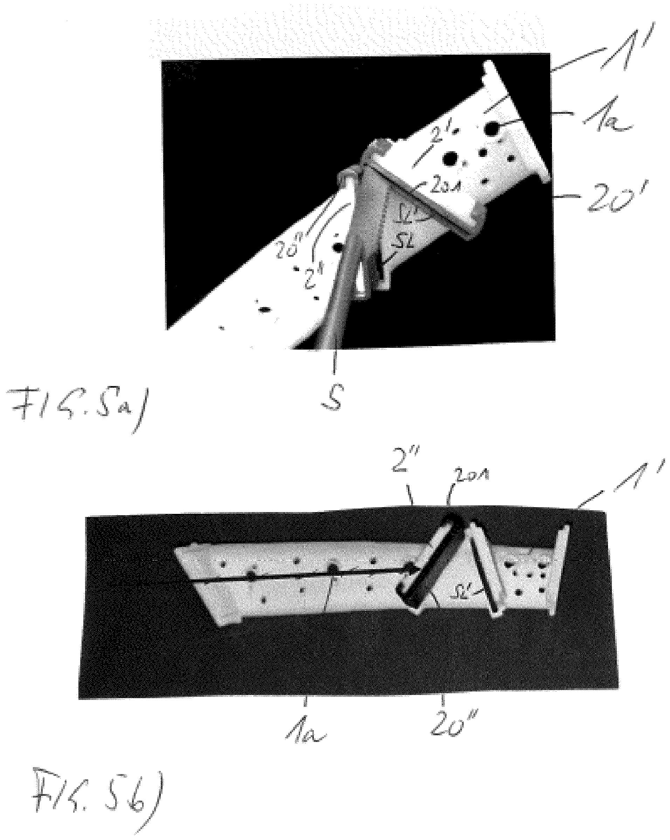

Fig. 5a), b) zeigen perspektivische Darstellungen einer Resektionsschablone gemäß einer zweiten Ausführungsform der vorliegenden Erfindung, und zwar Fig. 5a) eine ausschnittsweise perspektivische Darstellung mit eingestecktem Sägewerkzeug und Fig. 5b) eine perspektivische Darstellung mit nur einem aufgesteckten Schnittführungsaufsatz.

Die zweite Ausführungsform unterscheidet sich von der ersten Ausführungsform hinsichtlich der Ausbildung des Basisteils 1'. Das Basisteil 1' weist einen ersten und zweiten Befestigungsflansch 2', 2" auf, welche entlang eines jeweiligen ersten und zweiten Schlitzes SL, SL' des Basisteils 1' angeordnet sind. Dementsprechend vorgesehen sind ein erster und zweiter Schnittführungsaufsatz 20', 20", welche derart auf den ersten und zweiten Befestigungsflansch 2', 2" aufgesteckt sind, dass deren jeweiliger Sägeschlitz 201 entlang des ersten und zweiten Schlitzes SL, SL` des Basisteils 1' angeordnet ist.

In Fig. 5a) ist zusätzlich ein eingestecktes Sägewerkzeug S im Sägeschlitz 201 des ersten Schnittführungsaufsatzes 20' dargestellt.

Ansonsten ist die zweite Ausführungsform vollkommen analog zur ersten Ausführungsform ausgebildet.

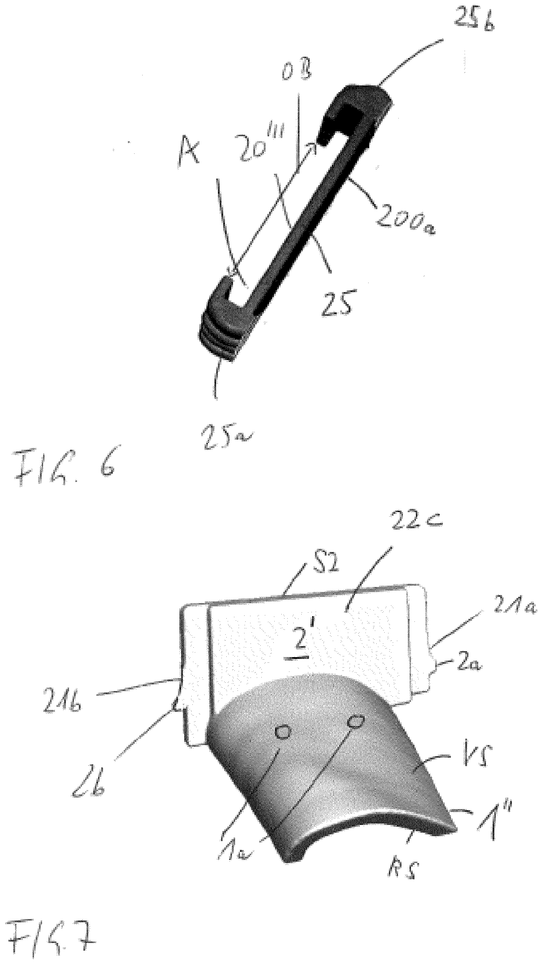

Fig. 6 zeigt eine perspektivische Darstellung eines Schnittführungsaufsatzes einer Resektionsschablone gemäß einer dritten Ausführungsform der vorliegenden Erfindung.

Die Darstellung von Fig. 6 ist analog zur Darstellung von Fig. 4 und unterscheidet sich von Fig. 4 hinsichtlich der Ausgestaltung des Schnittführungsaufsatzes.

Insbesondere weist der Schnittführungsaufsatz 20‴ gemäß Fig. 6 anstelle des Sägeschlitzes 201 einen Sägeanschlag 200a zum Führen eines Sägeschnittwerkzeugs aufweist, welcher bei aufgestecktem Schnittführungsaufsatz 20‴ entlang der Endkante K des Basisteils 1 oder entlang des jeweiligen Schlitzes SL, SL' des Basisteils 1' angeordnet ist.

Ansonsten ist die dritte Ausführungsform vollkommen analog zur ersten Ausführungsform ausgebildet.

Fig. 7 zeigt eine perspektivische Darstellung eines Basisteils einer Resektionsschablone gemäß einer vierten Ausführungsform der vorliegenden Erfindung.

Die Darstellung von Fig. 7 ist analog zur Darstellung von Fig. 2 bzw. Fig. 3 und unterscheidet sich von Fig. 2 bzw. Fig. 3 hinsichtlich der Ausgestaltung des Befestigungsflansches 2'.

Insbesondere weist der Befestigungsflansch 2' einen durchgehenden Anschlagsblock 22c auf, welcher derart angeordnet sind, dass er einen jeweiligen lateralen Anschlag für den ersten und zweiten Endumschlagbereich 25a, 25b bildet.

Ansonsten ist die vierte Ausführungsform vollkommen analog zur ersten Ausführungsform ausgebildet.

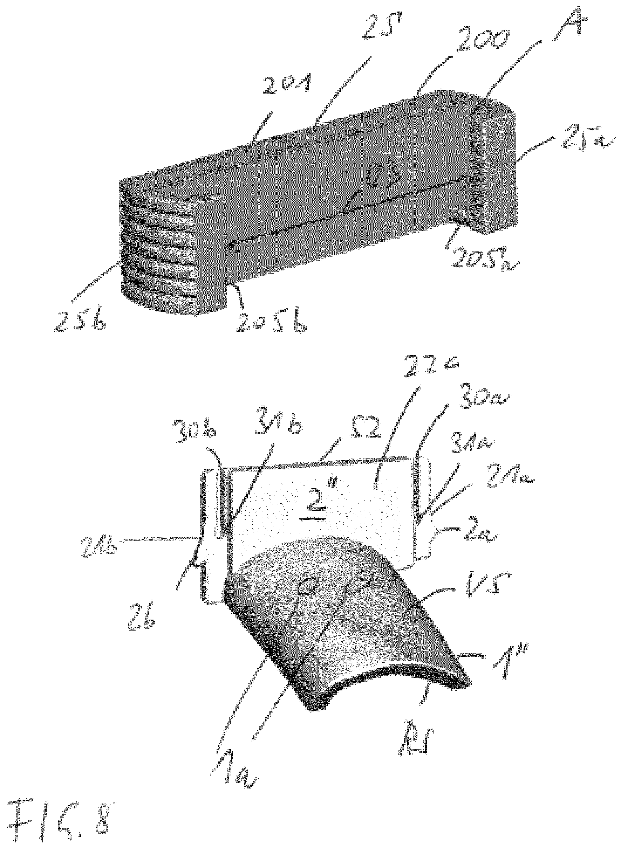

Fig. 8 zeigt eine ausschnittsweise perspektivische Explosionsansicht einer Resektionsschablone gemäß einer fünften Ausführungsform der vorliegenden Erfindung.

Die Darstellung von Fig. 8 ist analog zur Darstellung von Fig. 2 und unterscheidet sich von Fig. 2 hinsichtlich der Ausgestaltung des Schnittführungsaufsatzes 200 und des Befestigungsflansches 2" sowie der Kontur des Basisteils 1".

Der Befestigungsflansch 2" weist einen ersten Schlitz 30a und einen zweiten Schlitz 30b ausgehend von der der Vorderseite VS gegenüberliegenden Seite S2 des Befestigungsflansches 2" auf, welche in einem jeweils zugehörigen ersten Verbreiterungsbereich 31a und zweiten Verbreiterungsbereich 31b enden.

Der erste und zweite Endumschlagbereich 25a, 25b des Schnittführungsaufsatzes 200 weisen einen ersten und zweiten Verriegelungsbolzen 205a, 205b auf, welche durch den zugehörigen Schlitz 30a, 30b in den ersten und zweiten Verbreiterungsbereich 31a, 31b zum Verriegeln des Schnittführungsaufsatzes 200 einführbar sind.

Ansonsten ist die fünfte Ausführungsform vollkommen analog zur ersten Ausführungsform ausgebildet.

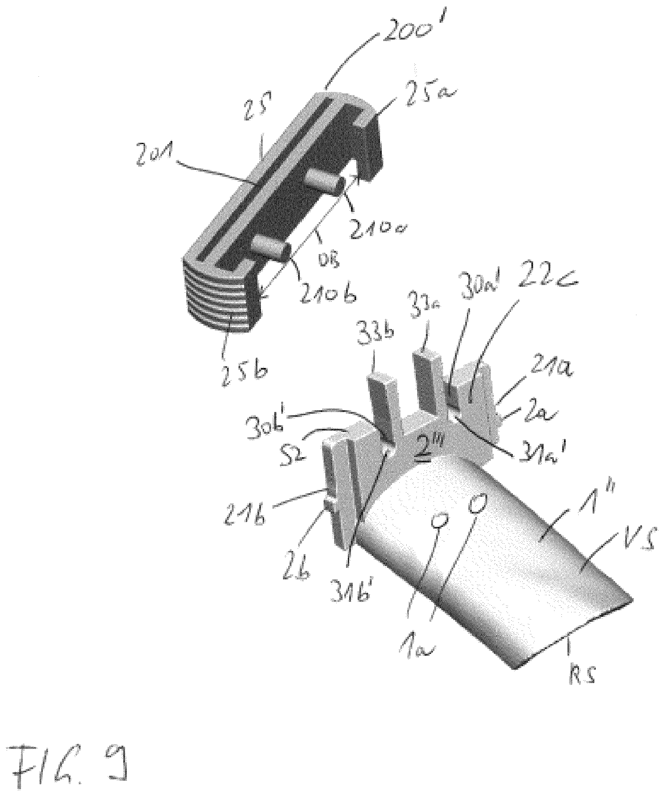

Fig. 9 zeigt eine ausschnittsweise perspektivische Explosionsansicht einer Resektionsschablone gemäß einer sechsten Ausführungsform der vorliegenden Erfindung.

Die Darstellung von Fig. 9 ist analog zur Darstellung von Fig. 2 und unterscheidet sich von Fig. 2 hinsichtlich der Ausgestaltung des Schnittführungsaufsatzes 200' und des Befestigungsflansches 2‴ sowie der Kontur des Basisteils 1".

Der Befestigungsflansch 2‴ weist einen ersten Schlitz 30a' und einen zweiten Schlitz 30b` ausgehend von der der Vorderseite VS gegenüberliegenden Seite S2 des Befestigungsflansches 2‴ auf, welche in einem jeweils zugehörigen ersten Verbreiterungsbereich 31a` und zweiten Verbreiterungsbereich 31b` enden.

Der geradlinige Bereich 25 des Schnittführungsaufsatzes 200' weist einen ersten und zweiten Verriegelungsbolzen 210a, 210b aufweist, welche durch den zugehörigen Schlitz 30a`, 30b` in den ersten und zweiten Verbreiterungsbereich 31a, 31b zum Verriegeln des Ringprofils einführbar sind.

Zusätzliche Führungsstege 33a, 33b auf der Seite S2 erleichtern das Aufstecken des Schnittführungsaufsatzes 200`. Eine Ausnutzung der Materialeigenschaften des Kunststoffs kann eine Verriegelung bewirken.

Ansonsten ist die sechste Ausführungsform vollkommen analog zur ersten Ausführungsform ausgebildet.

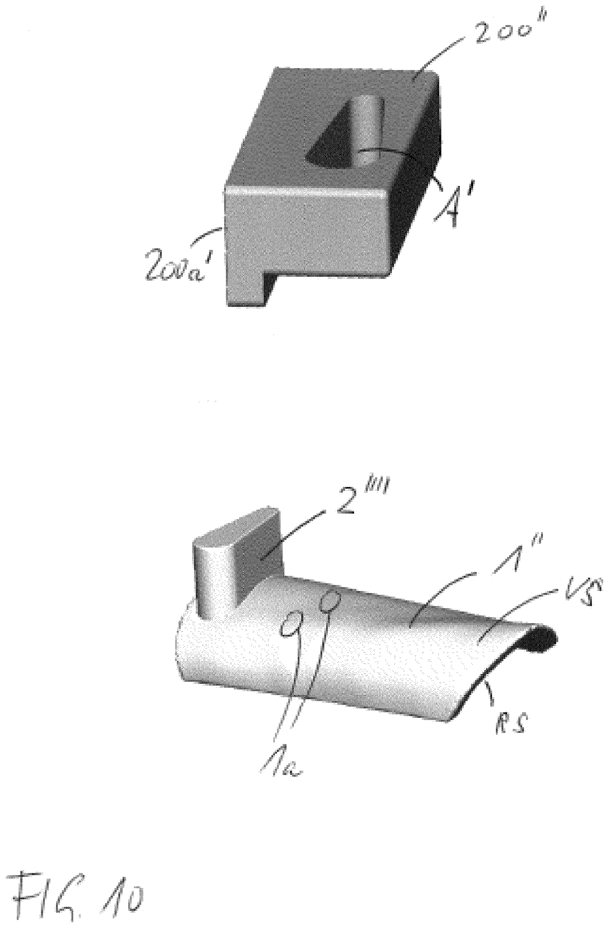

Fig. 10 zeigt eine ausschnittsweise perspektivische Explosionsansicht einer Resektionsschablone gemäß einer siebenten Ausführungsform der vorliegenden Erfindung.

Die Darstellung von Fig. 10 ist analog zur Darstellung von Fig. 2 und unterscheidet sich von Fig. 2 hinsichtlich der Ausgestaltung des Schnittführungsaufsatzes 200" und des Befestigungsflansches 2ʺʺ sowie der Kontour des Basisteils 1".

Insbesondere ist der Befestigungsflansch 2"" als Dornflanschflansch mit einem Dorn ausgebildet. Der Schnittführungsaufsatz 200" ist als Ringprofil ausgebildet, welches einen Dornaufnahmebereich A` und einen Sägeanschlag 200a` aufweist.

Der Dorn und der Dornaufnahmebereich weisen ein asymmetrisches Rundprofil auf, welches ein fehlerhaftes Aufstecken verhindert.

Ansonsten ist die siebente Ausführungsform vollkommen analog zur ersten Ausführungsform ausgebildet.

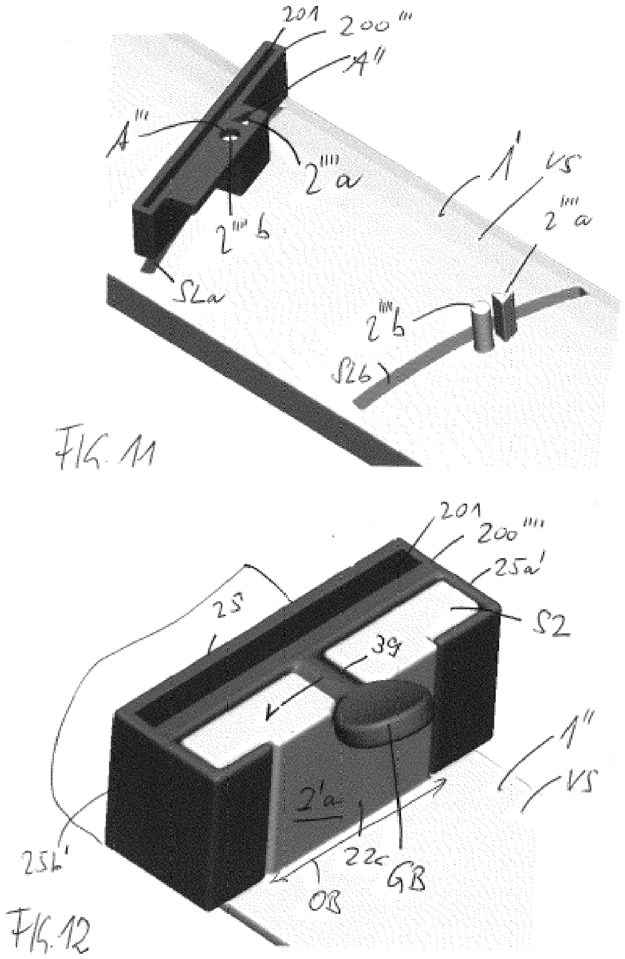

Fig. 11 zeigt eine ausschnittsweise perspektivische Darstellung einer Resektionsschablone gemäß einer achten Ausführungsform der vorliegenden Erfindung.

Die Darstellung von Fig. 11 ist analog zur Darstellung von Fig. 5b) und unterscheidet sich von Fig. 5b) hinsichtlich der Ausgestaltung des Schnittführungsaufsatzes 200‴ und der Befestigungsflansche 2""a, 2""b sowie der Kontur des Basisteils 1‴.

Insbesondere sind die Befestigungsflansche 2""a, 2""b als Dornflanschflansche? mit einem jeweiligen Dorn ausgebildet. Der Schnittführungsaufsatz 200‴ ist als in Profil ausgebildet, welches einen jeweiligen Dornaufnahmebereich A", A‴ und einen Sägeschlitz 201 aufweist. Der Sägeschlitz 201 ist entlang eines jeweiligen Schlitzes Sla, SLb des Basisteils 1‴ einführbar.

Die Dorne und die zugehörigen Dornaufnahmebereiche weisen ein jeweiliges asymmetrisches Rundprofil auf, welches ein fehlerhaftes Aufstecken verhindert.

Ansonsten ist die siebente Ausführungsform vollkommen analog zur zweiten Ausführungsform ausgebildet.

Fig. 12 zeigt eine ausschnittsweise perspektivische Darstellung einer Resektionsschablone gemäß einer neunten Ausführungsform der vorliegenden Erfindung.

Bei der neunten Ausführungsform weist der Befestigungsflansch 2'a keine Verbreiterungsbereiche und Endanschlagsbereiche auf. Als Endanschlag dient die Vorderseite des Basisteils 1", welches den Anschlagsblock 22c aufweist, welcher derart angeordnet sind, dass er einen jeweiligen lateralen Anschlag für den ersten und zweiten Endumschlagbereich 25a', 25b' des Schnittführungsaufsatzes 200"" bildet.

An der Vorderseite VS gegenüberliegenden Seite S2 des Befestigungsflansches 2'a ist eine Nut 39 vorgesehen, wobei der Schnittführungsaufsatz 200"" ausgehend von dem geradlinigen Bereich 25 einen Verbindungsbereich V aufweist, welcher in die Nut 39 einsetzbar ist, und wobei am dem geradlinigen Bereich 25 gegenüberliegenden Bereich des Verbindungsbereichs V ein Griffbereich GB vorgesehen ist.

Ansonsten ist die neunte Ausführungsform vollkommen analog zur vierten Ausführungsform ausgebildet.

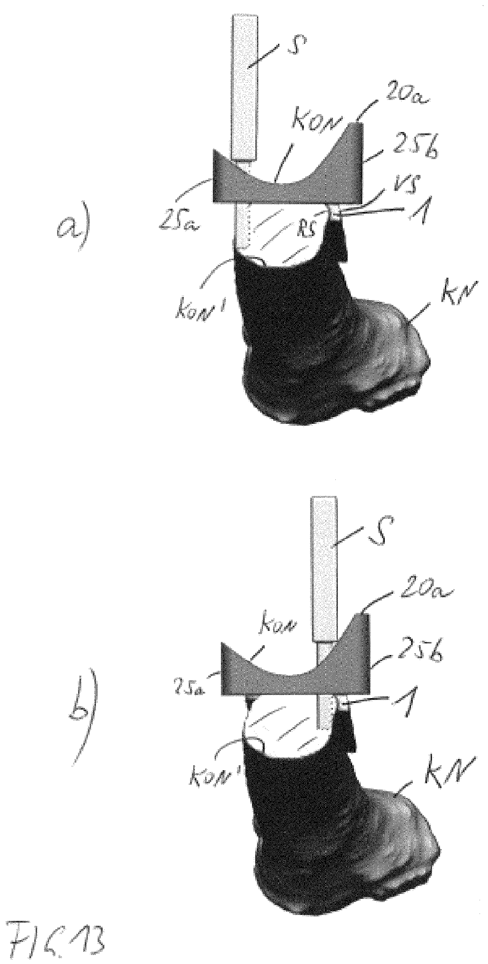

Fig. 13a),b) zeigen perspektivische Darstellungen einer Resektionsschablone gemäß einer zehnten Ausführungsform der vorliegenden Erfindung, und zwar Fig. 13a) eine ausschnittsweise perspektivische Darstellung mit eingestecktem Sägewerkzeug in einer ersten Position des Sägewerkzeugs und Fig. 13b) eine ausschnittsweise perspektivische Darstellung mit eingestecktem Sägewerkzeug in einer zweiten Position des Sägewerkzeugs.

Die zehnte Ausführungsform unterscheidet sich von der ersten Ausführungsform in der Ausgestaltung des Schnittführungsaufsatzes 20a. Insbesondere ist bei den zuvor beschriebenen Ausführungsformen der Schnittführungsaufsatz die Oberseite und die Unterseite des Schnittführungsaufsatzes planparallel angeordnet. Dies hat zur Folge, dass die Schnitttiefe des aufgesetzten Sägewerkzeuges über die gesamte laterale Erstreckung des Schnittführungsaufsatzes konstant ist.

Bei der zehnte Ausführungsform hingegen weist die Oberseite des Schnittführungsaufsatzes 20a eine gekrümmte Kontur KON auf, welche bewirkt, dass die Schnitttiefe des aufgesetzten Sägewerkzeuges über die laterale Erstreckung des Schnittführungsaufsatzes 20a variiiert.

Bei der zehnten Ausführungsform ist die Kontur KON der Oberseite des Schnittführungsaufsatzes 20a derart gekrümmt, dass sie eine Kontur KON' des darunterliegenden zu resektierenden Knochens KN nachbildet oder zumindest bereichsweise approximiert. Dadurch können Gewebestrukturen, welche den zu resektierenden Knochen KN umgeben, geschützt werden, da sie vom Sägewerkzeug S nicht erreichbar sind.

Ansonsten ist die zehnte Ausführungsform vollkommen analog zur ersten Ausführungsform ausgebildet.

Selbstverständlich kann eine derartige Ausgestaltung des Schnittführungsaufsatzes mit gekrümmter Kontur der Oberseite auch bei allen anderen zuvor beschriebenen Ausführungsformen realisiert werden.

Obwohl die vorliegende Erfindung anhand bevorzugter Ausführungsformen erläutert wurde, ist sie darauf nicht beschränkt, sondern insbesondere hinsichtlich der Geometrie und Materialien ihrer Bestandteile vielfältig modifizierbar.

## Patentansprüche

1. Resektionsschablone zur Resektion von einem Knochen mit:
einem plattenförmigen Basisteil (1; 1'; 1") mit einer Vorderseite (VS) und einer Rückseite (RS);
wobei die Rückseite (RS) eine Auflagefläche (10) zur Auflage auf dem Knochen aufweist;
wobei die Vorderseite (VS) einen sich von der Vorderseite (VS) weg erstreckenden Befestigungsflansch (2; 2'; 2'a; 2"; 2‴; 2""; 2""a, 2ʺʺb) aufweist, welcher benachbart zu einer Endkante (K) des Basisteils (1; 1'; 1") oder einem Schlitz (SL, SL'; SLa, SLb) des Basisteils (1; 1'; 1") angeordnet ist;
einem Schnittführungsaufsatz (20; 20', 20"; 20‴; 200; 200'; 200"; 200‴; 200""; 20a) , welcher von einer der Vorderseite (VS) gegenüberliegenden Seite (S2) des Befestigungsflansches (2; 2'; 2'a; 2"; 2‴; 2""; 2ʺʺa, 2ʺʺb) auf den Befestigungsflansch (2; 2'; 2'a; 2"; 2‴; 2""; 2ʺʺa, 2ʺʺb) aufsteckbar ist;
wobei der Schnittführungsaufsatz (20; 20', 20"; 20‴; 200; 200'; 200"; 200‴; 200""; 20a) einen Sägeschlitz (201) oder einen Sägeanschlag (200a, 200a') zum Führen eines Sägeschnittwerkzeugs aufweist, welcher bei aufgestecktem Schnittführungsaufsatz (20; 20', 20"; 20‴; 200; 200'; 200"; 200‴; 200""; 20a) entlang der Endkante (K) des Basisteils (1; 1'; 1") oder entlang des Schlitzes (SL, SL'; SLa, SLb) des Basisteils (1; 1'; 1") angeordnet ist.

2. Resektionsschablone nach Anspruch 1, wobei der Befestigungsflansch (2; 2'; 2'a; 2"; 2‴) als Plattenflansch ausgebildet ist.

3. Resektionsschablone nach Anspruch 2, wobei der Schnittführungsaufsatz (20; 20', 20"; 20‴; 200; 200'; 200"; 200‴; 200""; 20a) als Ringprofil ausgebildet ist, welches einen geradlinigen Bereich (25) aufweist, in dem der Sägeschlitz (201) oder an dem der Sägeanschlag (200a, 200a') angeordnet ist, wobei sich an dem ersten Ende des geradlinigen Bereichs (25) ein erster Endumschlagbereich (25a; 25a`) und an dem zweiten Ende des geradlinigen Bereichs (25) ein zweiter Endumschlagbereich (25b; 25b`) befindet, wobei der erste und zweite Endumschlagbereich (25a, 25b; 25a', 25b`) zusammen mit dem geradlinigen Bereich (25) einen Aufnahmebereich (A) für den Plattenflansch bilden, und wobei der erste und zweite Endumschlagbereich (25a, 25b; 25a', 25b`) derart ausgebildet sind, dass sie den Plattenflansch an dessen gegenüberliegenden Randbereichen umschließen.

4. Resektionsschablone nach Anspruch 3, wobei sich zwischen dem ersten und zweiten Endumschlagbereich (25a, 25b; 25a', 25b`) ein offener Bereich (OB) des Ringprofils befindet.

5. Resektionsschablone nach Anspruch 4, wobei der Plattenflansch einen ersten Anschlagssteg (22a) und einen zweiten Anschlagssteg (22b) aufweist, welche derart angeordnet sind, dass sie einen jeweiligen lateralen Anschlag für den ersten und zweiten Endumschlagbereich (25a, 25b) bilden.

6. Resektionsschablone nach Anspruch 4, wobei der Plattenflansch einen Anschlagsblock (22c) aufweist, welcher derart angeordnet ist, dass er einen jeweiligen lateralen Anschlag für den ersten und zweiten Endumschlagbereich (25a, 25b; 25a', 25b`) bildet.

7. Resektionsschablone nach einem der Ansprüche 2 bis 6, wobei der Plattenflansch an seinen gegenüberliegenden Kanten einen jeweiligen ersten und zweiten Verbreiterungsbereich (21a, 21b) aufweist, welcher sich in Richtung von der der Vorderseite (VS) gegenüberliegenden Seite (S2) des Plattenflansches zur Vorderseite (VS) hin verbreitert, so dass beim Aufstecken eine zunehmende Klemmwirkung auf das Ringprofil ausübbar ist.

8. Resektionsschablone nach einem der Ansprüche 2 bis 7, wobei der Plattenflansch an seinen gegenüberliegenden Kanten einen jeweiligen ersten und zweiten Endanschlagsbereich (2a, 2b) aufweist, welche die Aufstecktiefe des Ringprofils auf den Plattenflansch begrenzen.

9. Resektionsschablone nach einem der Ansprüche 3 bis 6, wobei der erste und zweite Endumschlagbereich (25a, 25b) einen jeweiligen Griffriefenbereich aufweisen.

10. Resektionsschablone nach einem der Ansprüche 3 bis 9, wobei der Plattenflansch einen ersten Schlitz (30a) und einen zweiten Schlitz (30b) ausgehend von der Vorderseite (VS) gegenüberliegenden Seite (S2) des Plattenflansches aufweist, welche in einem jeweils zugehörigen ersten Verbreiterungsbereich (31a) und zweiten Verbreiterungsbereich (31b) enden, und wobei der erste und zweite Endumschlagbereich (25a, 25b) einen ersten und zweiten Verriegelungsbolzen (205a, 205b) aufweisen, welche durch den zugehörigen Schlitz (30a, 30b) in den ersten und zweiten Verbreiterungsbereich (31a, 31b) zum Verriegeln des Ringprofils einführbar sind.

11. Resektionsschablone nach einem der Ansprüche 3 bis 9, wobei der Plattenflansch einen ersten Schlitz (30a`) und einen zweiten Schlitz (30b`) ausgehend von der Vorderseite (VS) gegenüberliegenden Seite (S2) des Plattenflansches aufweist, welche in einem jeweils zugehörigen ersten Verbreiterungsbereich (31a') und zweiten Verbreiterungsbereich (31b') enden, und wobei der geradlinige Bereich (25) einen ersten und zweiten Verriegelungsbolzen (210a, 210b) aufweist, welche durch den zugehörigen Schlitz (30a', 30b') in den ersten und zweiten Verbreiterungsbereich (31a, 31b) zum Verriegeln des Ringprofils einführbar sind.

12. Resektionsschablone nach Anspruch 1, wobei der Befestigungsflansch (2""; 2""a, 2""b) als Dornflanschflansch mit einem oder mehreren Dornen ausgebildet ist.

13. Resektionsschablone nach Anspruch 12, wobei der Schnittführungsaufsatz (20; 20', 20"; 20‴; 200; 200'; 200"; 200‴; 200""; 20a) als Ringprofil ausgebildet ist, welches einen oder mehrere Dornaufnahmebereiche (A`; A", A‴) entsprechend der Anzahl von Dornen des Dornflansches aufweist.

14. Resektionsschablone nach Anspruch 3, wobei der Plattenflansch an der Vorderseite (VS) gegenüberliegenden Seite (S2) des Plattenflansches eine Nut (39) aufweist, wobei Schnittführungsaufsatz (20; 20', 20"; 20‴; 200; 200'; 200"; 200‴; 200""; 20a) ausgehend von dem geradlinigen Bereich (25) einen Verbindungsbereich (V) aufweist, welcher in die Nut (39) einsetzbar ist, und wobei am dem geradlinigen Bereich (25) gegenüberliegenden Bereich des Verbindungsbereichs (V) ein Griffbereich (GB) vorgesehen ist.

15. Resektionsschablone nach einem der vorhergehenden Ansprüche, wobei das plattenförmigen Basisteil (1; 1'; 1") eine Mehrzahl von Durchgangslöchern (1a) aufweist, welche zur Aufnahme von Befestigungsmitteln, insbesondere Schrauben oder Nägeln, zur Befestigung an dem Knochen dienen.

16. Resektionsschablone nach einem der vorhergehenden Ansprüche, wobei das plattenförmige Basisteil (1; 1'; 1") mit dem Befestigungsflansch (2; 2'; 2'a; 2"; 2‴; 2ʺʺ; 2ʺʺa, 2ʺʺb) aus einem Kunststoffmaterial und der Schnittführungsaufsatz (20; 20', 20"; 20‴; 200; 200'; 200"; 200‴; 200""; 20a) aus einem metallischen Material gebildet ist.

17. Resektionsschablone nach einem der vorhergehenden Ansprüche, wobei der Schnittführungsaufsatz (20; 20', 20"; 20‴; 200; 200'; 200"; 200‴; 200ʺʺ) derart ausgebildet ist, dass die Oberseite und die Unterseite des Schnittführungsaufsatzes planparallel angeordnet sind, so dass die Schnitttiefe eines aufgesetzten Sägewerkzeuges über die gesamte laterale Erstreckung des Schnittführungsaufsatzes konstant ist.

18. Resektionsschablone nach einem der Ansprüche 1 bis 16, wobei der Schnittführungsaufsatz (20a) derart ausgebildet ist, dass die Oberseite des Schnittführungsaufsatzes (20a) eine gekrümmte Kontur (KON) aufweist, welche bewirkt, dass die Schnitttiefe eines aufgesetzten Sägewerkzeuges über die laterale Erstreckung des Schnittführungsaufsatzes (20a) variiiert.

19. Resektionsschablone nach Anspruch 18, wobei die gekrümmte Kontur (KON) der Oberseite des Schnittführungsaufsatzes (20a) derart gekrümmt ist, dass sie eine Kontur (KON') des darunterliegenden zu resektierenden Knochens (KN) nachbildet oder zumindest bereichsweise approximiert.
